# EUROPÄISCHE PATENTANMELDUNG

(11) **EP 4 834 290 A1**
(43) Veröffentlichungstag der Anmeldung: **04.11.2026**
(21) Anmeldenummer: 25173108.9
(22) Anmeldetag: 29.04.2025
(51) Int. Cl.: A61F 2/30, A61F 2/34

(54) **GIESSFORM FÜR GRATFREIE ACETABULUM-SPACER**

(71) Anmelder: Heraeus Medical GmbH, 61273 Wehrheim (DE)
(72) Erfinder: Vogt, Sebastian, 61273 Wehrheim (DE); Kluge, Thomas, 61273 Wehrheim (DE); Nassut, Roman, 61273 Wehrheim (DE)
(74) Vertreter: Heraeus IP

(57) **Zusammenfassung**

Die Erfindung betrifft eine Gießform (100) zur Herstellung eines Acetabulum-Spacers, aufweisend einen Grundkörper (110) mit einer gewölbten ersten Schale (111) und einer gewölbten zweiten Schale (112), einen ersten Steg (113) und einen zweiten Steg (114), einen Einfüllstutzen (120) und eine erste Durchführung (121, 131), wobei die erste Schale gemeinsam mit der zweiten Schale einen Hohlraum (115) definiert, wobei der erste Steg und der zweite Steg benachbart zueinander an dem Grundkörper angeordnet sind und gemeinsam einen Schnittbereich (116) des Grundkörpers definieren, wobei der Schnittbereich zwischen dem ersten Steg und dem zweiten Steg angeordnet ist, und wobei die erste Durchführung ausgebildet ist, den Austritt von überschüssigem Knochenzement (200) aus dem Hohlraum der Gießform zu ermöglichen.

## Beschreibung

### GEBIET DER ERFINDUNG

Die vorliegende Erfindung betrifft das Gebiet der Medizintechnik, insbesondere Vorrichtungen zur Herstellung medizinischer Implantate, und die damit herstellbaren medizinischen Implantate selbst. Weiterhin betrifft die Erfindung ein Herstellungsverfahren, in dem die erfindungsgemäßen Vorrichtungen zur Herstellung medizinischer Implantate verwendet werden.

### TECHNISCHER HINTERGRUND

Spacer aus Polymethylmethacrylat-Knochenzement, die ein oder mehrere Antibiotika enthalten können, werden mit Erfolg beim zweizeitigen septischen Wechsel von infizierten Gelenkendoprothesen verwendet. Die Spacer werden dazu nach der Entfernung der infizierten Gelenkendoprothese und nachfolgendem Debridement als temporäre Implantate für die Interimszeit bis zur Implantation der Revisionsgelenkendoprothese eingesetzt. In der Interimszeit können aus den Spacern ein oder mehrere Antibiotika freigesetzt werden, die zum Beruhigen der Infektion beitragen können. Die Spacer werden zum "Deadspace-Management" eingesetzt, d. h. der Behandlung von chirurgisch- oder infektionsbedingten Hohlräumen, und können eine Atrophie des Bandapparates und der Muskulatur verhindern.

Bei der zweizeitigen septischen Revision von Hüftgelenkendoprothesen werden der Prothesenschaft mit dem Prothesenkopf und die Gelenkpfanne in einer ersten OP entfernt. Als Hüftgelenkspacer finden in der Interimsphase dabei häufig aus Polymethlymethacrylat (PMMA) gefertigte Schäfte mit einem kugelförmigen oder pilzförmigen Kopf Verwendung. Die Köpfe besitzen einen so großen Durchmesser, dass diese direkt in das Acetabulum (Hüftgelenkpfanne) auf das Knochengewebe eingesetzt werden können. Problematisch ist jedoch, dass die Spacerköpfe während der Artikulation auf der Knochenoberfläche des Acetabulums reiben können.

Alternativ dazu wurden sog. Acetabulum-Cup-Spacer entwickelt, welche die Gelenkpfanne nachbilden. Bei diesen handelt es sich um schalenförmige Spacer mit einer konkaven Fläche zur Aufnahme des Spacerkopfs und einer konvexen Rückseite, die mit Polymethylmethacrylat-Knochenzementteig im Acetabulum verankert werden können.

In EP3761909EPB1 wird ein Acetabulum-Cup-Spacer vorgeschlagen, der halbkugelförmig ausgebildet ist. Auf der Außenseite erstrecken sich vom Rand des Spacers Aussparungen oder auch Stege, die zum Zentrum der der Halbkugel ausgerichtet sind.

In US12127944B2 wird eine zweiteilige Gießform vorgeschlagen, die aus einem konkaven und einem konvexen Teil bestehen. Der konkave Teil der Gießform wird mit dem konvexen Teil durch Verschraubung verbunden. Der konvexe Teil enthält eine Öffnung zum Einpressen von Polymethylmethacrylat-Knochenzement. Weiterhin sind röhrenförmige Entlüftungen am Rand des konvexen Teils angeordnet. An der Trennung der beiden Teile kann sich ein Grat am gegossenen Spacer ausbilden. Grate können sehr scharfkantig sein und können nur aufwendig mechanisch entfernt werden, insbesondere wenn diese eine Dicke von etwa 0,3 mm oder mehr haben. Solche größeren Grate können entweder mit Hilfe von Zangen abgebrochen oder mit Fräsern abgefräst werden, was für den medizinischen Anwender mühsam ist und eine gewünschte Formung des Spacers erschwert.

### KURZE BESCHREIBUNG DER ERFINDUNG

Eine Aufgabe der Erfindung ist die Bereitstellung einer Gießform, welche insbesondere für die intraoperative Herstellung von Acetabulum-Cup-Spacern aus Polymethylmethacrylat-Knochenzement verwendet werden kann. Die erfindungsgemäßen Gießformen sollen die Bildung von Graten an den Spacern möglichst vermeiden. Eine weitere Aufgabe der Erfindung ist die Vermeidung von Lufteinschlüssen in den hergestellten Spacern. Die Erfindung beschäftigt sich auch mit der Aufgabe, die Ausbildung eines Angusses möglichst zu vermeiden, oder zumindest den beim Abformen gebildeten Anguss möglichst klein und leicht entfernbar zu halten.

Zudem ist es eine Zielsetzung der Erfindung, die Entnahme des geformten Spacers aus der Gießform möglichst leicht, schnell und für den medizinischen Anwender sicher zu gestalten. Insbesondere soll die Gefahr von Schnittverletzungen vermieden werden, welche aufgrund von scharfkantigen Graten entstehen können. Eine weitere Gefahr von Schnittverletzungen kann dadurch entstehen, dass der medizinische Anwender beim Aufschneiden einer Gießform mit einem Skalpell abrutscht. In einigen Ausführungsformen ermöglicht die erfindungsgemäße Gießform daher ein sicheres und verletzungsfreies Aufschneiden der Gießform. Eine weitere Aufgabe der Erfindung ist auch die möglichst kostengünstige Herstellung der Gießform. Dies betrifft beispielsweise die Auswahl der für die Gießform verwendeten Materialien, und die möglichst einfache geometrische Ausgestaltung der Gießform.

Einzelne, mehrere oder alle diese Aufgaben werden durch die hierin beschriebenen Gießformen gelöst, insbesondere diejenigen, welche in den Ansprüchen beschrieben sind.

### BEVORZUGTE AUSFÜHRUNGSFORMEN

Ein Aspekt der Erfindung betrifft in einer ersten Ausführungsform eine Gießform zur Herstellung eines Acetabulum-Spacers, aufweisend einen Grundkörper mit einer gewölbten ersten Schale und einer gewölbten zweiten Schale, einen ersten Steg und einen zweiten Steg, einen Einfüllstutzen und eine erste Durchführung, wobei die erste Schale gemeinsam mit der zweiten Schale einen Hohlraum definiert, wobei der erste Steg und der zweite Steg benachbart zueinander an dem Grundkörper angeordnet sind und gemeinsam einen Schnittbereich des Grundkörpers definieren, wobei der Schnittbereich zwischen dem ersten Steg und dem zweiten Steg angeordnet ist, und wobei die erste Durchführung ausgebildet ist, den Austritt von überschüssigem Knochenzement aus dem Hohlraum der Gießform zu ermöglichen.

Eine zweite Ausführungsform betrifft eine Gießform gemäß der ersten oben genannten Ausführungsform, wobei der Grundkörper eine erste Wandstärke aufweist, welche dicker als eine zweite Wandstärke des Schnittbereichs ist.

Eine dritte Ausführungsform betrifft eine Gießform gemäß der ersten oder zweiten oben genannten Ausführungsform, wobei der erste Steg und der zweite Steg ausgebildet sind, ein Skalpell zum Durchtrennen der Gießform entlang des Schnittbereichs zu führen.

Eine vierte Ausführungsform betrifft eine Gießform nach einer der vorangehenden Ausführungsformen, wobei der erste Steg und/oder der zweite Steg eine Lasche aufweist, um ein manuelles Auseinanderziehen der ersten Schale und der zweiten Schale zu erleichtern.

Eine fünfte Ausführungsform betrifft eine Gießform nach einer der vorangehenden Ausführungsformen, wobei der erste Steg und/oder der zweite Steg sich um mindestens 2 mm, weiter bevorzugt mindestens 3, 4, 5 oder 6 mm, von dem Grundkörper nach außen erstreckt.

Eine sechste Ausführungsform betrifft eine Gießform nach einer der vorangehenden Ausführungsformen, wobei sich der erste Steg und/oder der zweite Steg sich umlaufend um mindestens die Hälfte eines Umfangs des Grundkörpers erstreckt.

Eine siebte Ausführungsform betrifft eine Gießform nach einer der vorangehenden Ausführungsformen, weiterhin aufweisend ein Mittel zum Abtrennen eines Angusses, wobei das Mittel bevorzugt ein bewegliches Element aufweist, wobei das bewegliche Element weiter bevorzugt ausgewählt ist aus einer Durchführung und einem Vorsprung.

Eine achte Ausführungsform betrifft eine Gießform nach einer der vorangehenden Ausführungsformen, weiterhin aufweisend einen Stopfen zum Verschließen des Einfüllstutzens, wobei die erste Durchführung bevorzugt in dem Stopfen angeordnet ist.

Eine neunte Ausführungsform betrifft eine Gießform gemäß der achten Ausführungsform, wobei die Gießform ausgebildet und eingerichtet ist, durch eine Drehbewegung des Stopfens relativ zum Einfüllstutzen einen Anguss abzutrennen.

Eine zehnte Ausführungsform betrifft eine Gießform gemäß der achten oder neunten Ausführungsform, wobei der Stopfen einen ersten Vorsprung zur Abtrennung eines Angusses aufweist, wobei der erste Vorsprung bevorzugt an der ersten Durchführung angeordnet ist.

Eine elfte Ausführungsform betrifft eine Gießform nach Ausführungsform zehn, wobei der erste Vorsprung eine angeschrägte Schneidkante aufweist.

Eine zwölfte Ausführungsform betrifft eine Gießform nach einer der vorangehenden Ausführungsformen, wobei der Einfüllstutzen einen zweiten Vorsprung zur Abtrennung eines Angusses aufweist, wobei die Gießform bevorzugt weiterhin einen ersten Vorsprung gemäß der zehnten Ausführungsform aufweist, und wobei die Gießform bevorzugt ausgebildet und eingerichtet ist, durch eine Drehbewegung des Stopfens relativ zum Einfüllstutzen einen Anguss abzutrennen, wenn der erste Vorsprung relativ zu dem zweiten Vorsprung gedreht wird.

Eine dreizehnte Ausführungsform betrifft eine Gießform nach einer der vorangehenden Ausführungsformen acht bis zwölf, wobei die erste Durchführung exzentrisch in dem Stopfen angeordnet ist.

Eine vierzehnte Ausführungsform betrifft eine Gießform nach einer der vorangehenden Ausführungsformen acht bis dreizehn, wobei der Einfüllstutzen weiterhin eine zweite Durchführung aufweist, wobei die Gießform ausgebildet und eingerichtet ist, in einer ersten Drehposition des Stopfens die erste Durchführung entlang einer gemeinsamen Achse mit der zweiten Durchführung anzuordnen, und in einer zweiten Drehposition des Stopfens die erste Durchführung außerhalb einer gemeinsamen Achse mit der zweiten Durchführung anzuordnen.

Eine fünfzehnte Ausführungsform betrifft eine Gießform nach einer der vorangehenden Ausführungsformen, weiterhin aufweisend ein von außerhalb der Gießform zugängliches Bedienelement, welches ausgebildet und eingerichtet ist, einen Anguss abzutrennen.

Eine sechzehnte Ausführungsform betrifft eine Gießform nach einer der vorangehenden Ausführungsformen, weiterhin aufweisend eine Entlüftungsöffnung, wobei die Entlüftungsöffnung bevorzugt zwischen der ersten Schale und der zweiten Schale angeordnet ist.

Ein weiterer Aspekt betrifft die Verwendung einer Gießform nach einer der vorangehenden Ausführungsformen zur Herstellung eines Acetabulum-Spacers.

Ein weiterer Aspekt betrifft ein Verfahren zur Herstellung eines Acetabulum-Spacers, aufweisend die nachfolgenden Schritte,
Bereitstellen einer Gießform nach einem der Ausführungsformen 1 bis 16;
Befüllen der Gießform mit Knochenzement;
Härten des Knochenzements in der Gießform;
gegebenenfalls Entfernen eines aus dem Knochenzement gebildeten Angusses;
Auftrennen des Grundkörpers der Gießform im Schnittbereich (116);
Trennen der ersten Schale von der zweiten Schale (112); und
Entnahme des hergestellten Acetabulum-Spacers aus der aufgetrennten Gießform.

### KURZE BESCHREIBUNG DER ZEICHNUNGEN

**Figur 1** zeigt beispielhaft eine erste Ausführungsform der Erfindung in einer Querschnittsansicht.
**Figur 2** zeigt beispielhaft eine Ausführungsform eines Stopfens in einer Querschnittsansicht.
**Figur 3** zeigt beispielhaft eine weitere Ausführungsform der Erfindung in einer Querschnittsansicht.
**Figur 4** zeigt beispielhaft eine weitere Ausführungsform der Erfindung.
**Figur 5** zeigt beispielhaft eine erfindungsgemäße Gießform, welche mit Knochenzement befüllt ist.
**Figur 6** zeigt beispielhaft eine erfindungsgemäße Gießform, die aufgetrennt ist.
**Figur 7** zeigt einen Ausschnitt einer Gießform, die einen Einfüllstutzen mit einem Bedienelement aufweist, in einer ersten Position.
**Figur 8** zeigt einen Ausschnitt einer Gießform, die einen Einfüllstutzen mit einem Bedienelement aufweist, in einer ersten Position als Aufsicht.
**Figur 9** zeigt einen Ausschnitt einer Gießform, die einen Einfüllstutzen mit einem Bedienelement aufweist, in einer zweiten Position.
**Figur 10** zeigt einen Ausschnitt einer Gießform, die einen Einfüllstutzen mit einem Bedienelement aufweist, in einer zweiten Position als Aufsicht.
**Figur 11** zeigt eine erfindungsgemäße Gießform und einer Austragsvorrichtung für Knochenzement, welche zur Verbindung mit der Gießform angeordnet ist.

### AUSFÜHRLICHE BESCHREIBUNG

Zu den hierin beschriebenen Ausführungsformen, deren Elemente ein bestimmtes Merkmal (z.B. ein Material) "aufweisen", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Gießformen und Spacern anwendbar sind, und umgekehrt. Lediglich aus Gründen der knapperen Darstellung werden alle diese erwogenen Kombinationen nicht in allen Fällen explizit aufgeführt.

Ein erster Aspekt der Erfindung betrifft eine Gießform zur Herstellung von Spacern. Die erfindungsgemäße Gießform ist insbesondere zur Herstellung eines Acetabulum-Spacers geeignet. Ein solcher Spacer wird hierin auch als Acetabulum-Cup-Spacer bezeichnet. Ein solcher Spacer bildet die Form der Gelenkpfanne eines Hüftgelenks nach. Acetabulum-Spacer weisen üblicherweise eine schalenförmige Form mit einer konkaven Fläche zur Aufnahme eines Gelenkkopfs oder Spacerkopfs auf. Weiterhin weist ein Acetabulum-Spacer üblicherweise eine konvexe Rückseite auf, die mit Knochenzement im Acetabulum angebracht werden kann.

Die erfindungsgemäße Gießform weist einen Grundkörper auf, der durch eine gewölbte erste Schale und eine gewölbte zweite Schale gebildet ist. Am Übergang von der ersten Schale zur zweiten Schale sind ein erster Steg und ein zweiter Steg angeordnet. Weiterhin weist die Gießform einen Einfüllstutzen auf. Dieser Einfüllstutzen kann zur Befüllung der Gießform mit Knochenzement dienen. Die erste Schale definiert gemeinsam mit der zweiten Schale einen Hohlraum, der zur Befüllung mit Knochenzement vorgesehen ist. Der erste Steg und der zweite Steg definieren gemeinsam einen Schnittbereich des Grundkörpers. An dieser Stelle kann die Gießform aufgetrennt werden, beispielsweise mithilfe eines Skalpells, um einen in der Gießform hergestellten Spacer zu entnehmen. Der Schnittbereich ist zwischen dem ersten Steg und dem zweiten Steg angeordnet. Die Gießform weist weiterhin eine erste Durchführung auf, welche den Austritt von überschüssigem Knochenzement aus dem Hohlraum der Gießform ermöglicht.

Eine Ausführungsform der Erfindung betrifft demnach eine Gießform zur Herstellung eines Acetabulum-Spacers, aufweisend einen Grundkörper mit einer gewölbten ersten Schale und einer gewölbten zweiten Schale, einen ersten Steg und einen zweiten Steg, einen Einfüllstutzen und eine erste Durchführung, wobei die erste Schale gemeinsam mit der zweiten Schale einen Hohlraum definiert, wobei der erste Steg und der zweite Steg benachbart zueinander an dem Grundkörper angeordnet sind und gemeinsam einen Schnittbereich des Grundkörpers definieren, wobei der Schnittbereich zwischen dem ersten Steg und dem zweiten Steg angeordnet ist, und wobei die erste Durchführung ausgebildet ist, den Austritt von überschüssigem Knochenzement aus dem Hohlraum der Gießform zu ermöglichen.

Die Gießform weist einen Hohlraum auf. Dieser Hohlraum wird im Wesentlichen durch die erste Schale und die zweite Schale ausgebildet. Der Hohlraum kann eine glatte Oberfläche aufweisen. In einer Ausführungsform weisen sowohl die erste Schale als auch die zweite Schale eine vollständig glatte innere Oberfläche auf. In einigen Ausführungsformen ist die Gießform ausgebildet, Spacer mit Oberflächenstrukturen herzustellen. Hierzu kann eine innere Oberfläche der ersten Schale und/oder eine innere Oberfläche der zweiten Schale entsprechende Strukturen aufweisen. Beispiele für solche Strukturen umfassen Stege, Noppen und Vertiefungen. Eine solche strukturierte Oberfläche kann beispielsweise die Befestigung eines Acetabulum-Spacers in einem Hüftgelenk des Patienten erleichtern. Daher kann es vorteilhaft sein, wenn insbesondere die erste Schale eine innere Oberfläche mit einer solchen Struktur aufweist.

Die Gießform kann einen Kunststoff aufweisen. Die Gießform kann vollständig oder teilweise aus Kunststoff bestehen. Dies ermöglicht eine kostengünstige Herstellung, beispielsweise durch ein Spritzgussverfahren. Beispielsweise können die erste Schale und die zweite Schale jeweils getrennt voneinander im Spritzgussverfahren aus Kunststoff hergestellt werden, und danach miteinander verschweißt bzw. verschmolzen werden. In einigen Ausführungsformen ist die Gießform einteilig ausgebildet. In einigen Ausführungen ist die Gießform frei von Trennungen, welche zu Graten in dem darin hergestellten Spacer führen können. In einigen Ausführungsformen ist die erste Schale fest mit der zweiten Schale verbunden. In einigen Ausführungsformen ist die erste Schale nahtlos mit der zweiten Schale verbunden. Insbesondere kann der Schnittbereich der Gießform einen Kunststoff aufweisen. Dies ermöglicht ein leichtes Auftrennen der Gießform entlang des Schnittbereichs. In einigen Ausführungsformen ist der Kunststoff ein thermoplastischer Kunststoff. Beispiele für verwendbare Kunststoffe umfassen Polyethylen und Polypropylen. Besonders bevorzugt sind Kunststoffe mit geringer Dichte wie z.B. LD-PE. Bevorzugt weist der Kunststoff eine Dichte von weniger als 0,94 g/cm³ bei 1 bar und 25 °C auf.

Der Schnittbereich verläuft bevorzugt umlaufend am Grundkörper der Gießform. In einer Ausführungsform erstreckt sich der Schnittbereich mindestens entlang der Hälfte des Umfangs des Grundkörpers der Gießform. Der Grundkörper der Gießform weist bevorzugt einen im Wesentlichen kreisförmigen Querschnitt auf. In Versuchen zeigte sich, dass ein ungefähr hälftiges Auftrennen, d. h. ein Auftrennen der Gießform entlang der Hälfte des Umfangs des Grundkörpers der Gießform, in der Regel ausreichend ist, um die Gießform im Wesentlichen vollständig zu öffnen und den darin hergestellten Spacer zu entnehmen.

Um das Auftrennen der Gießform entlang des Schnittbereichs zu erleichtern, kann es vorteilhaft sein, den Schnittbereich mit einer reduzierten Wandstärke auszugestalten. In einer Ausführungsform weist der Grundkörper eine erste Wandstärke auf, und der Schnittbereich weist eine zweite Wandstärke auf, wobei die erste Wandstärke dicker als die zweite Wandstärke ist. Dies bedeutet, dass der Schnittbereich im Vergleich zum Rest des Grundkörpers der Gießform eine reduzierte Wandstärke aufweisen kann.

In einer Ausführungsform sind der erste Steg und der zweite Steg ausgebildet, ein Skalpell zum Durchtrennen der Gießform entlang des Schnittbereichs zu führen. Hierzu kann ein Benutzer der Gießform ein Skalpell zwischen dem ersten Steg und dem zweiten Steg entlang des Schnittbereichs führen, um die Gießform aufzuschneiden. Der erste Steg und der zweite Steg können hierbei verhindern, dass der Benutzer beim Führen des Skalpells abrutscht, und sich dadurch verletzt. Ähnlich wie oben dargestellt, können sich der erste Steg der zweite Steg umlaufend um den Grundkörper der Gießform erstrecken. Bevorzugt erstrecken sich der erste Steg und der zweite Steg mindestens um die Hälfte des Umfangs des Grundkörpers.

Um die Gießform leichter öffnen zu können, können der erste Steg und/oder der zweite Steg eine Lasche aufweisen. In einer Ausführungsform weist der erste Steg eine Lasche auf. In einer Ausführungsform weist der zweite Steg eine Lasche auf. Bevorzugt weisen sowohl der erste Steg als auch der zweite Steg jeweils eine Lasche auf. Dies erleichtert ein manuelles Auseinanderziehen der ersten Schale und der zweiten Schale. Hierzu kann ein Benutzer der Gießform die Laschen mit der Hand greifen und dadurch die erste Schale von der zweiten Schale trennen, wobei er die beiden Laschen auseinanderzieht. Danach lässt sich der in der Gießform hergestellte Spacer leicht entnehmen.

In einer Ausführungsform erstreckt sich der erste Steg und/oder der zweite Steg um mindestens 2 mm von dem Grundkörper nach außen. Dies kann es einem Benutzer der Vorrichtung erleichtern, ein Skalpell zum Durchtrennen der Gießform entlang des Schnittbereichs zu führen. In einer Ausführungsform erstreckt sich der erste Steg um mindestens 2 mm von dem Grundkörper nach außen. In einer Ausführungsform erstreckt sich der zweite Steg um mindestens 2 mm von dem Grundkörper nach außen. In einer Ausführungsform erstreckt sich sowohl der erste Steg als auch der zweite Steg um mindestens 2 mm von dem Grundkörper nach außen. In einigen Ausführungsformen erstreckt sich der erste Steg und/oder der zweite Steg um mindestens 3, 4, 5 oder 6 mm von dem Grundkörper nach außen.

In einigen Ausführungsformen erstreckt sich der erste Steg und/oder der zweite Steg umlaufend um mindestens die Hälfte eines Umfangs des Grundkörpers. Dies kann es einem Benutzer der Vorrichtung ermöglichen, ein Skalpell sicher entlang des Schnittbereichs um mindestens die Hälfte des Umfangs des Grundkörpers zu führen, um die Gießform so weit aufzutrennen, dass sich ein darin hergestellter Spacer leicht aus der Gießform entnehmen lässt. In einer Ausführungsform erstreckt sich der Schnittbereich umlaufend mindestens um die Hälfte eines Umfangs des Grundkörpers.

In einigen Ausführungsformen weist der Schnittbereich eine verringerte Wandstärke auf. In einigen Ausführungsformen weist der Schnittbereich eine Wandstärke von höchstens 1 mm, bevorzugt höchstens 0,5 mm oder höchstens 0,4 mm auf. Dies kann es einem Benutzer der Gießform ermöglichen, die Gießform am Schnittbereich mit geringem Kraftaufwand aufzutrennen. Dies kann einen Beitrag zur Vermeidung von Verletzungen leisten, dabei geringem Kraftaufwand auch ein Abrutschen des Skalpells weniger wahrscheinlich ist. Zudem verringert sich die Wahrscheinlichkeit, dass beim Aufschneiden der Gießform der darin enthaltene Spacer und/oder das Skalpell beschädigt wird.

In einigen Ausführungsformen weist die Gießform ein Mittel zum Abtrennen eines Angusses auf. Das Mittel zum Abtrennen eines Angusses kann bevorzugt ein bewegliches Element aufweisen. Das bewegliche Element kann eine Durchführung aufweisen, insbesondere eine hierin beschriebene erste Durchführung oder eine hierin beschriebene zweite Durchführung. Das bewegliche Element kann einen Vorsprung aufweisen, insbesondere einen hierin beschriebenen ersten Vorsprung oder einen hierin beschriebenen zweiten Vorsprung.

In einigen Ausführungsformen weist die Gießform einen Stopfen auf. Der Stopfen kann zum Verschließen des Einfüllstutzens ausgebildet sein. Der Stopfen weist bevorzugt einen Durchmesser auf, welcher geringer als einen Durchmesser des Einfüllstutzens ist. Der Stopfen ist bevorzugt drehbar in den Einfüllstutzen einbringbar. Dies bedeutet, dass der Stopfen in einem Zustand, in welcher der Stopfen den Einfüllstutzen verschließt, weiterhin gedreht werden kann, ohne die Gießform zu öffnen. Die hierin beschriebene erste Durchführung kann bevorzugt in dem Stopfen angeordnet sein. Demnach verschließt der Stopfen den Einfüllstutzen nicht vollständig hermetisch, sondern erlaubt das Herausfließen von überschüssigem Knochenzement aus der Gießform, wenn der Stopfen in den Einfüllstutzen gedrückt wird.

Die Gießform weist einen Einfüllstutzen auf. Der Einfüllstutzen kann eine im Wesentlichen zylindrische Form aufweisen. Der Einfüllstutzen kann durch einen Stopfen der Gießform verschließbar sein. Der Einfüllstutzen kann einen Anschlag aufweisen, um die Eindringtiefe des Stopfens in den Einfüllstutzen zu begrenzen. Dieser Anschlag kann zweckmäßig als Verjüngung des Einfüllstutzens ausgestaltet sein. Demnach kann der Einfüllstutzen einen Hals aufweisen, welcher als Verjüngung einer zylindrischen Grundform des Einfüllstutzens ausgebildet ist.

Die hierin beschriebene erste Durchführung definiert bevorzugt einen Zugang, durch welchen der Hohlraum der Gießform mit Knochenzement befüllbar ist. Dieser Zugang bildet bevorzugt die einzige Position, an welcher der in der Gießform hergestellte Spacer einen Anguss mit einer Breite von mehr als 1 mm aufweist. Durch die hierin beschriebenen Ausgestaltungen kann dieser Anguss minimiert oder dessen Bildung sogar vollständig vermieden werden, oder der Anguss kann durch entsprechende Ausgestaltungen der Gießform abgetrennt werden. Hierdurch kann ein Spacer ohne Anguss oder mit nur geringfügigem Anguss hergestellt werden, wobei ein solcher geringfügiger Anguss leicht entfernbar ist.

In einer Ausführungsform ist die Gießform ausgebildet und eingerichtet, mithilfe eines beweglichen Elements der Gießform einen Anguss abzutrennen. Das bewegliche Element kann eine Durchführung oder einen Vorsprung aufweisen. Das bewegliche Element kann in einem Stopfen der Gießform oder im Einfüllstutzen der Gießform angeordnet sein.

In einer Ausführung ist die Gießform ausgebildet und eingerichtet, durch eine Drehbewegung des Stopfens relativ zum Einfüllstutzen einen Anguss abzutrennen. Diese Funktion kann durch unterschiedliche Ausgestaltungen erzielt werden, wie hierin beschrieben. Beispielsweise kann der Stopfen eine im wesentlichen zylindrische Form aufweisen. Die erste Durchführung kann exzentrisch in dem Stopfen angeordnet sein, d. h. die erste Durchführung kann versetzt zu einer Mittelachse des Stopfens angeordnet sein. Wenn bei einer solchen Ausführungsform der Gießform in einem Zustand, in welchem die Gießform über den Einfüllstutzen mit Knochenzement befüllt ist, der Stopfen gedreht wird, kann hierdurch der Anguss abgetrennt werden. Dies bedeutet, dass der Knochenzement im Hohlraum der Gießform nicht mehr mit dem Knochenzement in der ersten Durchführung verbunden ist.

Weiterhin kann der Stopfen einen ersten Vorsprung zur Abtrennung eines Angusses aufweisen. In einer Ausführungsform ist der erste Vorsprung in einem Innenraum des Stopfens angeordnet. Der erste Vorsprung ist bevorzugt an der ersten Durchführung angeordnet. Der erste Vorsprung kann dazu dienen, Druck auf den Knochenzement auszuüben, um den Anguss wie oben erläutert abzutrennen. Weiterhin kann der erste Vorsprung eine angeschrägte Schneidkante aufweisen. Hierdurch kann der erste Vorsprung nach Funktionsweise eines Messers den Anguss leichter durchtrennen. Die Schneidkante verkleinert die Fläche des ersten Vorsprungs, welche beim Drehen des Stopfens gegen den Anguss gedrückt werden kann. Hierdurch wird die Kraft, die mit der Drehung des Stopfens verbunden ist, über eine geringere Fläche auf den Anguss ausgeübt.

Hierin bezeichnen eine "erste Durchführung" und ein "erster Vorsprung" bevorzugt solche Elemente, die in einem Stopfen der Gießform angeordnet sind. Hingegen bezeichnen eine "zweite Durchführung" und ein "zweiter Vorsprung" bevorzugt solche Elemente, die in dem Einfüllstützen der Gießform angeordnet sind.

Die hierin im Zusammenhang mit dem ersten Vorsprung genannten Merkmale und Funktionen können jeweils in entsprechender Weise auch für den nachfolgend erläuterten zweiten

Vorsprung Anwendung finden. Dabei werden jegliche Ausführungsformen erwogen, bei denen ein entsprechendes Merkmal entweder nur bei dem ersten Vorsprung, nur bei dem zweiten Vorsprung, oder sowohl beim ersten Vorsprung als auch beim zweiten Vorsprung vorhanden ist. Beispielsweise können sowohl der erste Vorsprung als auch der zweite Vorsprung jeweils eine angeschrägte Schneidkante aufweisen. Diese beiden Schneidkanten können zusammenwirken, sodass die beiden Schneidkanten sich nach Art einer Schere gegeneinander bewegen und gemeinsam die Abtrennung eines Angusses bewirken können.

In entsprechender Weise kann die Gießform eine erste Durchführung und eine zweite Durchführung aufweisen, und jeweils ausgebildet und in der Gießform angeordnet sind, um in einer ersten Konfiguration der Gießform linear aneinander ausgerichtet zu werden, und in einer zweiten Konfiguration der Gießform versetzt zueinander ausgerichtet zu werden. Hierdurch kann einerseits die Durchlässigkeit des Einfüllstutzens für Knochenzement gesteuert werden, und andererseits ein Anguss abgetrennt werden. Hierzu können die erste Durchführung und/oder die zweite Durchführung exzentrisch angeordnet sein. Weiterhin können der erste Vorsprung und/oder der zweite Vorsprung exzentrisch angeordnet sein. "Exzentrisch" bezieht sich im Zusammenhang mit der ersten Durchführung und/oder des ersten Vorsprungs auf eine zentrale Mittelachse des Stopfens, und im Zusammenhang mit der zweiten Durchführung und/oder des zweiten Vorsprungs auf eine zentrale Mittelachse des Einfüllstutzens.

In einer Ausführungsform weist die Gießform einen zweiten Vorsprung zur Abtrennung eines Angusses auf. In einigen Ausführungsformen kann die Gießform, wie oben beschrieben, einen ersten Vorsprung aufweisen, und zusätzlich einen solchen zweiten Vorsprung aufweisen. In einer Ausführungsform ist die Gießform ausgebildet und eingerichtet, durch eine Drehbewegung des Stopfens relativ zum Einfüllstutzen einen Anguss abzutrennen, wenn der erste Vorsprung relativ zu dem zweiten Vorsprung gedreht wird. Hierzu kann der erste Vorsprung, wie oben beschrieben, bevorzugt an dem Stopfen angeordnet sein. Der erste Vorsprung kann an dem Stopfen, bevorzugt in dem Stopfen angeordnet sein. Der zweite Vorsprung kann an dem Stopfen, bevorzugt in dem Stopfen angeordnet sein. Der erste Vorsprung kann exzentrisch an dem Stopfen angeordnet sein. Der zweite Vorsprung kann exzentrisch an dem Einfüllstutzen angeordnet sein.

Weiterhin können kann der Stopfen und/oder der Einfüllstutzen jeweils eine Vielzahl von Durchführungen aufweisen. In einer Ausführungsform weist der Stopfen zwei erste Durchführungen auf. In einer Ausführungsform weist der Einfüllstutzen zwei zweite Durchführungen auf.

In einer Ausführungsform weist der Stopfen eine Vielzahl von Durchführungen auf, die punktspiegel-symmetrisch ausgebildet und angeordnet sind. In einer Ausführungsform weist der Einfüllstutzen eine Vielzahl von Durchführungen auf, die punktspiegel-symmetrisch ausgebildet und angeordnet sind. In einer Ausführungsform weisen sowohl der Stopfen als auch der Einfüllstutzen jeweils eine Vielzahl von Durchführungen auf, die punktspiegel-symmetrisch ausgebildet und angeordnet sind.

In einer Ausführungsform weist der Einfüllstutzen eine Vielzahl von Durchführungen auf, wobei die Fläche der Vielzahl von Durchführungen insgesamt mindestens 30 % der Innenquerschnittsfläche des Einfüllstutzens beträgt.

In einer Ausführungsform weist der Stopfen eine Vielzahl von Durchführungen auf, wobei die Fläche der Vielzahl von Durchführungen insgesamt mindestens 30 % der Innenquerschnittsfläche des Stopfens beträgt. Die "Innenquerschnittsfläche" leitet sich dabei jeweils von der kleinsten lichten Weite des jeweiligen Elements ab.

In einigen Ausführungsformen sind die Vielzahl von Durchführungen des Stopfens und die Vielzahl von Durchführungen des Einfüllstutzens jeweils gleichartig zueinander ausgestaltet. Beispielsweise kann der Stopfen zwei Durchführungen aufweisen, welche im Querschnitt jeweils die Form eines Dreiecks aufweisen, wobei sich die Spitze der beiden Dreiecksquerschnitte berührt, oder nahezu berührt. Der Einfüllstutzen kann ebenfalls zweiten Durchführungen aufweisen, welche im Querschnitt jeweils die Form eines Dreiecks aufweisen, wobei sich die Spitze der beiden Dreiecksquerschnitte berührt, oder nahezu berührt. Durch eine Drehung des Stopfens können somit in einer ersten Konfiguration der Gießform die oben genannten Durchführungen aneinander ausgerichtet werden, sodass Knochenzement durch den Einfüllstutzen in den Hohlraum der Gießform eingebracht werden kann. Durch eine weitere Drehung des Stopfens können die oben genannten Durchführungen versetzt zueinander ausgerichtet werden, sodass kein Knochenzement durch den Einfüllstutzen mehr gelangen kann. Somit kann ein Benutzer der Vorrichtung durch Drehung des Stopfens zwischen einem geöffneten Zustand der Gießform und einem geschlossenen Zustand der Gießform wechseln. Gleichzeitig kann hierdurch auch ein Anguss entfernt werden, wie hierin beschrieben. Hierzu können gegebenenfalls ein erster Vorsprung und ein zweiter Vorsprung gegeneinander gedreht werden.

In anderen Ausführungsformen der Erfindung kann eine solche Funktion auch ohne einen Stopfen erzielt werden. In einer Ausführungsform weist die Gießform ein Bedienelement auf, welches ausgebildet und eingerichtet ist, eine hierin beschriebene Durchführung und/oder einen hierin beschriebenen Vorsprung zu verschieben, um einen Anguss abzutrennen. In einer Ausführungsform weist die Gießform ein von außerhalb der Gießform zugängliches Bedienelement auf, welches ausgebildet und eingerichtet ist, einen Anguss abzutrennen.

Das Bedienelement kann gleichermaßen ausgebildet und eingerichtet sein, zwischen einem geöffneten Zustand der Gießform und einem geschlossenen Zustand der Gießform zu wechseln, ähnlich wie oben beschrieben. Das Bedienelement kann bevorzugt an dem Einfüllstutzen angeordnet sein. Das Bedienelement ist bevorzugt von außen zugänglich, sodass ein Benutzer der Gießform das Bedienelement von außen betätigen kann, um die Funktion des Bedienelements auszuführen. In einer Ausführungsform ist das Bedienelement ausgebildet und eingerichtet, durch Verbindung einer Austragsvorrichtung für Knochenzement mit einer erfindungsgemäßen Gießform die Gießform in eine erste Konfiguration zu bringen, in welcher Knochenzement in die Gießform eingebracht werden kann. Hierzu wird mithilfe des Bedienelements wie hierin beschrieben eine Durchführung so angeordnet, dass Knochenzement durch den Einfüllstutzen in die Gießform gelangen kann.

In einigen Ausführungsformen weist der Einfüllstutzen einen zweiten Vorsprung zur Abtrennung eines Angusses auf. In einigen Ausführungsformen weist der Stopfen einen ersten Vorsprung zur Abtrennung eines Angusses auf, und der Einfüllstutzen weist einen zweiten Vorsprung zur Abtrennung eines Angusses auf. In einigen Ausführungsformen ist die Gießform ausgebildet und eingerichtet, durch eine Drehbewegung des Stopfens relativ zum Einfüllstutzen einen Anguss abzutrennen, wenn der erste Vorsprung relativ zu dem zweiten Vorsprung gedreht wird.

In einigen Ausführungsformen weist der Einfüllstutzen eine zweite Durchführung auf. In einigen Ausführungsformen weist der Stopfen eine erste Durchführung auf, und der Einfüllstutzen weist eine zweite Durchführung auf, wobei die Gießform ausgebildet und eingerichtet ist, in einer ersten Drehposition des Stopfens die erste Durchführung entlang einer gemeinsamen Achse mit der zweiten Durchführung anzuordnen, und in einer zweiten Drehposition des Stopfens die erste Durchführung versetzt zu einer solchen Achse der zweiten Durchführung anzuordnen.

In einigen Ausführungsformen ist die Gießform ausgebildet und eingerichtet, durch Drehung eines Stopfens der Gießform oder durch Drehung eines Bedienelements zwischen einem geöffneten Zustand der Gießform und einem geschlossenen Zustand der Gießform zu wechseln.

In einigen Ausführungsformen ist die Gießform ausgebildet und eingerichtet, durch Drehung eines Stopfens der Gießform oder durch Drehung eines Bedienelements einen Anguss abzutrennen.

Die Gießform kann weiterhin ein oder mehrere Entlüftungsöffnungen aufweisen. Eine solche Entlüftungsöffnung kann es ermöglichen, den Hohlraum der Gießform vollständig oder nahezu vollständig mit Knochenzement zu befüllen. Hierdurch können Luftblaseneinschlüsse bei dem in der Gießform herstellbaren Spacer vermieden werden. Die Entlüftungsöffnung kann bevorzugt zwischen der ersten Schale und der zweiten Schale angeordnet sein. Es können mehrere Entlüftungsöffnungen zwischen der ersten und der zweiten Schale angeordnet sein. Es können mehrere Entlüftungsöffnungen in regelmäßigen Abständen umlaufend an der Gießform zwischen der ersten oder zweiten Schale angeordnet sein. Die Entlüftungsöffnung kann gegenüber des Einfüllstutzens angeordnet sein. Es können mehrere Entlüftungsöffnungen gegenüber des Einfüllstutzens angeordnet sein. Die Entlüftungsöffnung oder die Entlüftungsöffnungen weisen bevorzugt einen Durchmesser von weniger als 1,0 mm auf.

Mit "Knochenzement" wird hierin sowohl ein "Knochenzementteig" bezeichnet, d.h. eine formbare Masse, die zu einem festen, nicht mehr verformbaren Material aushärtbar ist, als auch der entsprechende ausgehärtete Knochenzement bezeichnet.

In entsprechender Weise bezieht sich der Begriff "Anguss" hierin auf verformbaren oder ausgehärteten Knochenzement, der eine üblicherweise unerwünschte Struktur an einem in der Gießform hergestellten Spacer bildet. Ein solcher Anguss kann sich an einer Zuleitung zum Hohlraum der Gießform ausbilden, insbesondere im Bereich der hierin beschriebenen ersten Durchführung und/oder der hierin beschriebenen zweiten Durchführung.

Handelsübliche Knochenzemente werden in vielen Fällen als Kit angeboten, welches eine flüssige und eine feste Komponente enthält. Durch Mischen der flüssigen mit der festen Komponente kann der Anwender einen Knochenzementteig herstellen, der für eine begrenzte Zeit leicht verformbar ist. Bereits kurze Zeit nach dem Mischen wird der Knochenzementteig klebfrei, d. h. er haftet bei leichter Berührung mit einem Handschuh nicht mehr an diesem an (wie definiert in ISO 5833:2002). In diesem Zustand kann ein Knochenzementteig bevorzugt in eine erfindungsgemäße Gießform zur Herstellung von Spacern eingefüllt werden, beispielsweise mithilfe eines Rührspatels, oder bevorzugt mithilfe einer geeigneten Austragsvorrichtung. Handelsübliche Knochenzemente werden häufig auf Basis von Polymethylmethacrylat (PMMA) angeboten. Die erfindungsgemäße Gießform kann bevorzugt mit einem solchen PMMA-Knochenzement verwendet werden. Es ist jedoch grundsätzlich auch möglich, andere Arten von Knochenzementen zu verwenden.

Die Erfindung betrifft weiterhin die Verwendung einer hierin beschriebenen Gießform zur Herstellung eines Acetabulum-Spacers. Hierzu kann die Gießform wie hierin beschrieben mit Knochenzement befüllt werden, wobei der Knochenzement in der Gießform einer Härtung unterzogen werden kann, um einen festen Acetabulum-Spacer herzustellen. Die Gießform kann anschließend wie hierin beschrieben geöffnet werden, und der hergestellte Acetabulum-Spacer kann aus der Gießform entnommen werden.

Die Erfindung betrifft weiterhin ein Verfahren zur Herstellung eines Acetabulum-Spacers. Hierzu kann eine hierin beschriebene Gießform verwendet werden. Das Verfahren kann die folgenden Schritte aufweisen:
Bereitstellen einer hierin beschriebenen Gießform;
Befüllen der Gießform mit Knochenzement;
Härten des Knochenzements in der Gießform;
Auftrennen des Grundkörpers der Gießform im Schnittbereich;
Trennen der ersten Schale von der zweiten Schale; und
Entnahme des hergestellten Acetabulum-Spacers aus der aufgetrennten Gießform.

Das Verfahren kann weiterhin gegebenenfalls das Entfernen eines aus dem Knochenzement gebildeten Angusses umfassen, wie hierin beschrieben.

Die oben dargestellten Schritte des Verfahrens können grundsätzlich in jeder Reihenfolge erfolgen. In einer Ausführungsform erfolgt die Durchführung des Verfahrens in der oben dargestellten Reihenfolge.

Ein weiterer Aspekt der Erfindung ist ein Spacer, der mithilfe einer hierin beschriebenen Gießform herstellbar ist, oder hergestellt ist.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

### FIGUREN

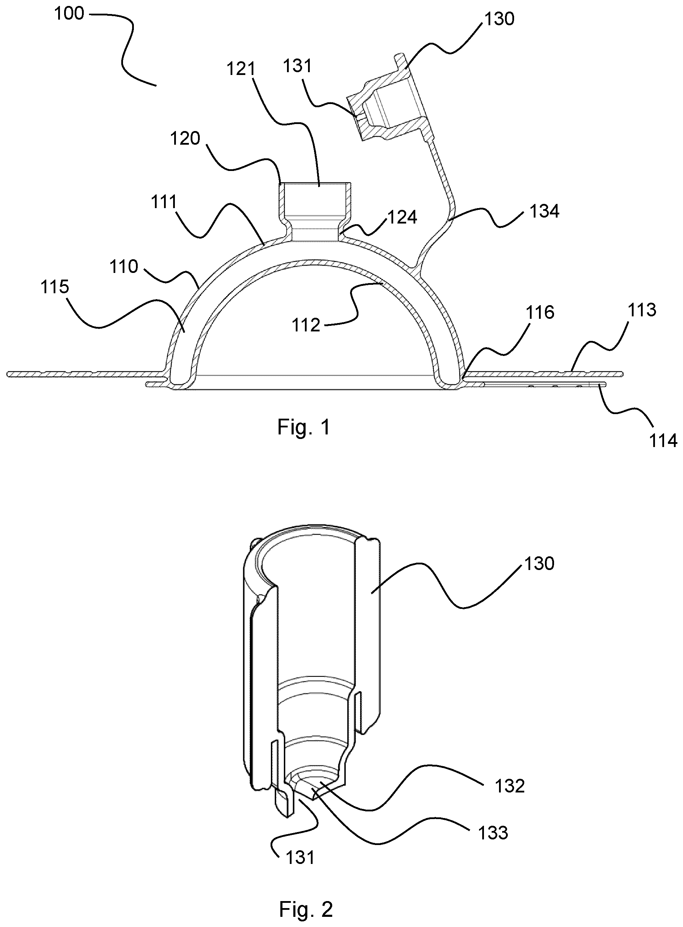

**Figur 1** zeigt beispielhaft eine erste Ausführungsform der Erfindung in einer Querschnittsansicht. Eine Gießform **100** weist einen Grundkörper **110** auf, welcher eine erste gewölbte Schale **111** und eine zweite gewölbte Schale **112** aufweist. Die erste Schale **111** weist eine konvex geformte Außenoberfläche auf. Die zweite Schale **112** weist eine konkav geformte Außenoberfläche auf. Die erste Schale **111** bildet gemeinsam mit der zweiten Schale **112** einen inneren Hohlraum **115.** Dieser Hohlraum **115** ist zur Befüllung mit Knochenzement vorgesehen, und entspricht der Form des herzustellenden Acetabulum-Spacers. Zur Befüllung des Hohlraums **115** weist die Gießform **100** weiterhin einen Einfüllstutzen **120** auf, welcher an der ersten Schale **111** angeordnet ist. Der Einfüllstutzen **120** weist hier eine im wesentlichen zylindrische Form auf, um eine formschlüssige Verbindung mit einer Austragsvorrichtung zu ermöglichen. Der Einfüllstutzen **120** weist hier einen Hals **124** auf, welcher als Verjüngung der zylindrischen Grundform des Einfüllstutzens **120** ausgebildet ist. Der Hals **124** verhindert ein zu tiefes Eindringen einer Austragsvorrichtung in die Gießform, sodass eine Störung des Abformprozesses verhindert werden kann. Der Hals **124** bildet einen Anschlag, sodass eine Austragsvorrichtung nicht über den Einfüllstutzen **120** hinaus bis in den Hohlraum **115** zwischen der ersten Schale **111** und der zweiten Schale **112** vordringen kann. Die Gießform **100** weist weiterhin einen ersten Steg **113** und einen zweiten Steg **114** auf, die benachbart zueinander am Grundkörper **110** angeordnet sind, und sich vom Grundkörper **110** aus nach außen erstrecken. Die beiden Stege weisen jeweils Laschen auf, so dass sie jeweils von einem Benutzer mit der Hand gefasst werden können. Der erste Steg **113** und der zweite Steg **114** definieren gemeinsam einen dazwischenliegenden Schnittbereich **116** der Gießform. Dieser Schnittbereich **116** ist mit einer Vergleich zum Rest des Grundkörpers geringeren Wandstärke ausgestaltet, sodass der Schnittbereich **116** leicht mithilfe eines Skalpells aufgetrennt werden kann. Der erste Steg **113** und der zweite Steg **114** können dabei zur Führung der Bewegung des Skalpells dienen. Hierdurch kann ein Abrutschen des Skalpells vermieden werden, und somit eine verletzungssichere Öffnung der Gießform **100** ermöglicht werden. Die Gießform **100** weist weiterhin einen Stopfen **130** auf, der zum Verschließen des Einfüllstutzens **120** ausgebildet ist. Der Stopfen **130** ist hier über ein Gelenk **134** mit dem Grundkörper **110** der Gießform verbunden. Hierdurch wird der Stopfen **130** stets an der Gießform **100** gehalten, und es kann somit beispielsweise vermieden werden, dass der Stopfen **130** herunterfallen und dadurch beispielsweise unsteril werden könnte. Der Stopfen **130** weist eine erste Durchführung **131** auf, die zur Herstellung einer Verbindung mit dem Hohlraum **115** ausgebildet ist. Mithilfe der ersten Durchführung **131** kann überschüssiger Knochenzement aus der Gießform **100** herausfließen, wenn die Gießform nach dem Befüllen mit Knochenzement mit dem Stopfen **130** verschlossen wird. Die erste Durchführung **130** weist einen geringen Durchmesser von beispielsweise 2 mm oder weniger auf, sodass nur ein sehr kleiner Anguss aus Knochenzement entsteht, der leicht entfernt werden kann. In der hier gezeigten Ausführungsform ist die erste Durchführung **131** konzentrisch, d. h. entlang einer Mittelachse des Stopfens **130,** angeordnet.

**Figur 2** zeigt beispielhaft eine Ausführungsform eines Stopfens **130** in einer Querschnittsansicht. Der Stopfen **130** weist eine erste Durchführung **131** und einen ersten Vorsprung **132** auf. Der Vorsprung **132** weist am Rand des Vorsprungs, welcher in Richtung der ersten Durchführung **131** weist, eine angeschrägte Form auf, welche eine Schneidkante **133** bildet. Die erste Durchführung **131** ist hier nicht zentriert an der Mittelachse des Stopfens **130** angeordnet, sondern ist in einer exzentrischen Position des Stopfens angeordnet. Diese Ausgestaltung des Stopfens **130** ermöglicht es, durch eine Drehung des Stopfens **130** im Einfüllstutzen **120** einen Anguss aus Knochenzement abzutrennen. Hierbei durchtrennt die sich bewegende Schneidkante **133** des ersten Vorsprung **132** den Anguss. Bei dieser Ausführungsform kann es sinnvoll sein, einen Stopfen **130** ohne Gelenk **134** zu verwenden, um eine freie Drehbarkeit des Stopfens **130** zu ermöglichen.

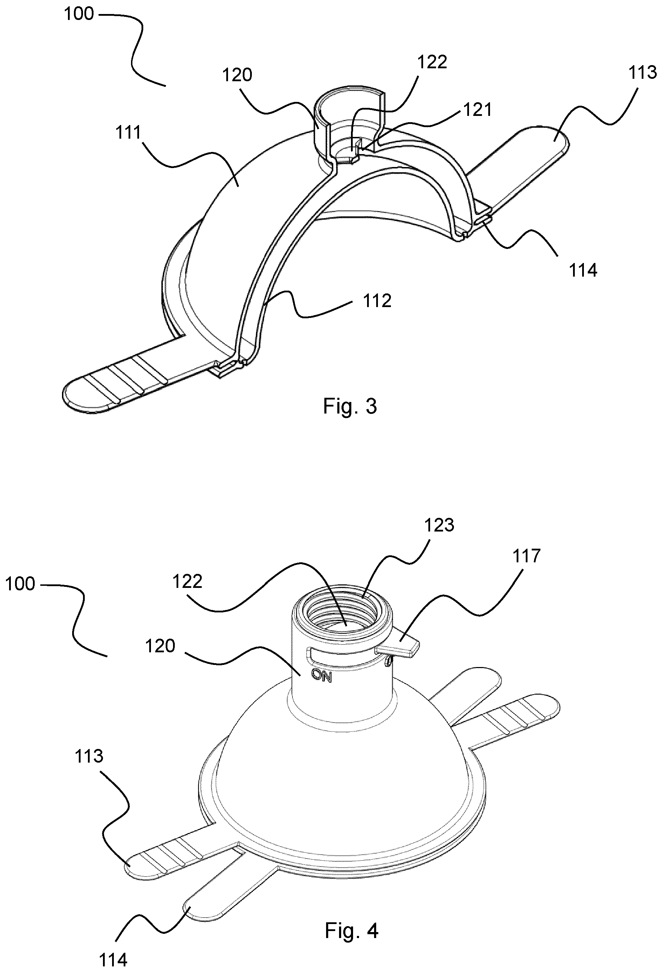

**Figur 3** zeigt beispielhaft eine weitere Ausführungsform der Erfindung in einer Querschnittsansicht. Eine Gießform **100** weist eine erste Schale **111** und eine zweite Schale **112** auf, die miteinander verbunden sind. An der ersten Schale **111** ist ein Einfüllstutzen **120** angeordnet, welcher eine zweite Durchführung **121** und einen zweiten Vorsprung **122** aufweist. Die zweite Durchführung **121** und der zweite Vorsprung **122** können alternativ oder zusätzlich zu einer ersten Durchführung **131** und einem ersten Vorsprung **132** vorhanden sein. Die zweite Durchführung **121** und der zweite Vorsprung **122** begrenzen die Querschnittsfläche des in die Gießform aufzunehmenden Knochenzements. Hierdurch kann an der entsprechenden Stelle ein dünnerer Anguss aus Knochenzement gebildet werden, welcher leicht entfernt werden kann.

**Figur 4** zeigt beispielhaft eine weitere Ausführungsform der Erfindung. Die hier gezeigte Gießform **100** weist einen ersten Steg **113** und einen zweiten Steg **114** auf, die jeweils Laschen aufweisen, und somit leicht von einem Benutzer mit der Hand gefasst werden können, um nach dem Aufschneiden der Gießform die erste Schale **111** von der zweiten Schale **112** zu trennen, und den darin geformten Spacer aus der Gießform zu entnehmen. Die hier gezeigte Ausführungsform weist einen Einfüllstutzen **120** mit einem zweiten Vorsprung **122** auf, der operativ mit einem Bedienelement **117** verbunden ist. Das Bedienelement **117** ist von außen zugänglich, und ermöglicht eine Bewegung des Vorsprungs **122** im innen liegenden Teil des Einfüllstutzens **120.** Die Bewegung des Vorsprungs **122** ermöglicht es, einen Anguss von einem in der Gießform geformten Spacer abzutrennen. Der Einfüllstutzen **120** weist weiterhin ein Innengewinde **123** zur Verbindung mit einer Austragsvorrichtung für Knochenzement auf. Wie hierin erläutert, kann das Bedienelement **117** ausgebildet und eingerichtet sein, bei der Verbindung mit einer Austragsvorrichtung das Bedienelement **117** in eine erste Position zu bringen, welche die Aufnahme von Knochenzement in die Gießform erlaubt. Weiterhin kann das Bedienelement **117** ausgebildet und eingerichtet sein, durch Verschieben des Bedienelements **117** in eine zweite Position einen Anguss aus Knochenzement abzutrennen. In einigen Ausführungsformen verschließt der Vorsprung **122** in einer solchen zweiten Position des Bedienelements **117** den Einfüllstutzen **120,** sodass kein Knochenzement mehr durch den Einfüllstutzen **120** fließen kann.

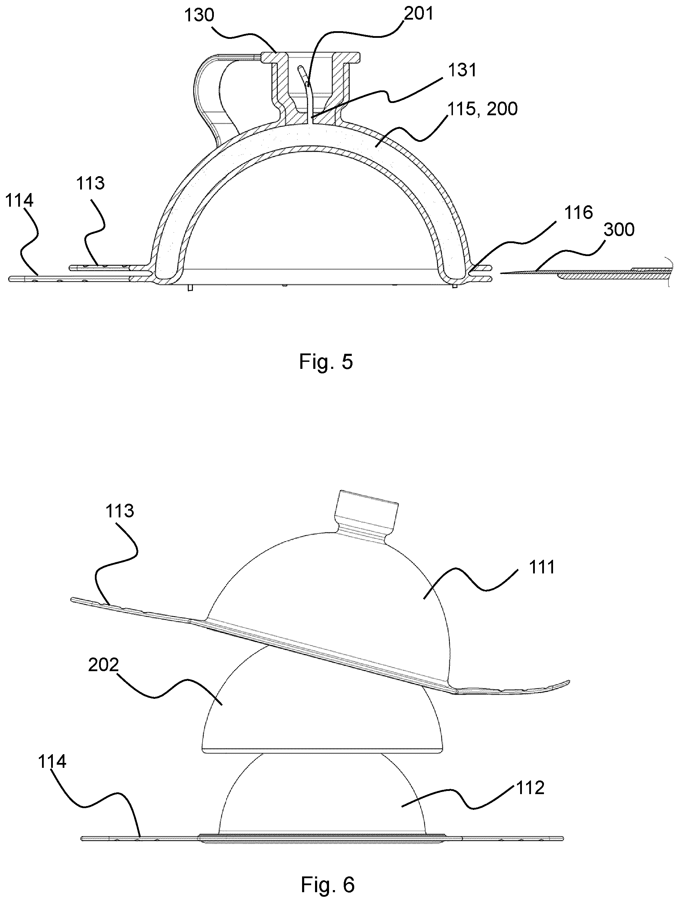

**Figur 5** zeigt beispielhaft eine erfindungsgemäße Gießform, welche mit Knochenzement **200** befüllt ist. Der Hohlraum **115** ist vollständig mit Knochenzement **200** befüllt. Durch Verschließen der Gießform mit dem Stopfen **130** entsteht ein leichter Überdruck des aufgenommenen Knochenzements **200,** sodass überschüssiger Knochenzement **200** durch die erste Durchführung **131** aus dem Hohlraum **115** nach außen abgegeben werden kann. Hierdurch bildet sich ein dünner Anguss **201,** dessen Dicke im Wesentlichen durch den Durchmesser der ersten Durchführung **131** bestimmt ist. Dieser dünner Anguss **201** kann nach dem Härten des Knochenzements **200** leicht von dem in der Gießform hergestellten Acetabulum-Spacer entfernt werden. Zur Entnahme des Spacers aus der Gießform ist es erforderlich, die Gießform zu öffnen. Zu diesem Zweck kann die Gießform nach dem Härten des Knochenzements entlang des umlaufenden Schnittbereichs **116** mithilfe eines Skalpells **300** aufgetrennt werden. Dies ermöglicht es, die erste Schale **111** von der zweiten Schale **112** zu trennen. Hierbei können weiterhin der erste Steg **113** und der zweite Steg **114,** welche jeweils eine Lasche aufweisen, von einem Benutzer mit der Hand auseinandergezogen werden, um die erste Schale **111** von der zweiten Schale **112** zu trennen.

**Figur 6** zeigt beispielhaft eine erfindungsgemäße Gießform, bei welcher die erste Schale **111** wie zuvor beschrieben von der zweiten Schale **112** getrennt worden ist. Durch Greifen des ersten Stegs **113** und des zweiten Stegs **114** können die beiden Schalen manuell auseinandergezogen werden, um den Spacer **202** aus der Gießform zu entnehmen.

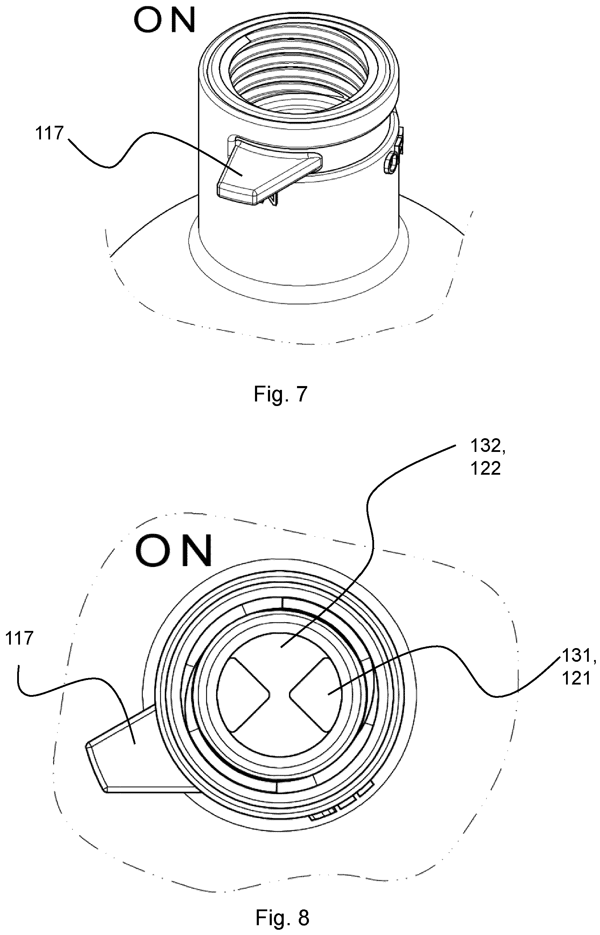

**Figur 7** zeigt einen Ausschnitt einer Gießform, die wie in Bezug auf Figur 4 erläutert einen Einfüllstutzen **120** mit einem Bedienelement **117** aufweist. Das Bedienelement **117** befindet sich hier in einer ersten Position, welche den Durchfluss von Knochenzement durch den Einfüllstutzen **120** erlaubt. Diese erste Position des Bedienelements **107** ist mit einer Beschriftung "ON" gekennzeichnet, um einem Benutzer der Gießform zu erkennen zu geben, dass in dieser Konfiguration der Gießform Knochenzement in die Gießform eingefüllt werden kann.

**Figur 8** zeigt einen Ausschnitt einer Gießform gemäß Figur 7, wobei sich das Bedienelement **117** in einer ersten Position befindet. Hierbei weist die Gießform eine erste Durchführung **131** und eine zweite Durchführung **121,** welche in der ersten Position des Bedienelements **117** entlang einer gemeinsamen Achse angeordnet sind, und somit den Durchfluss von Knochenzement durch den Einfüllstutzen **120** erlauben. Weiterhin weist die Gießform einen ersten Vorsprung **132** und einen zweiten Vorsprung **122** auf, die ebenfalls in der ersten Position des Bedienelements **117** entlang einer gemeinsamen Achse aneinander ausgerichtet sind.

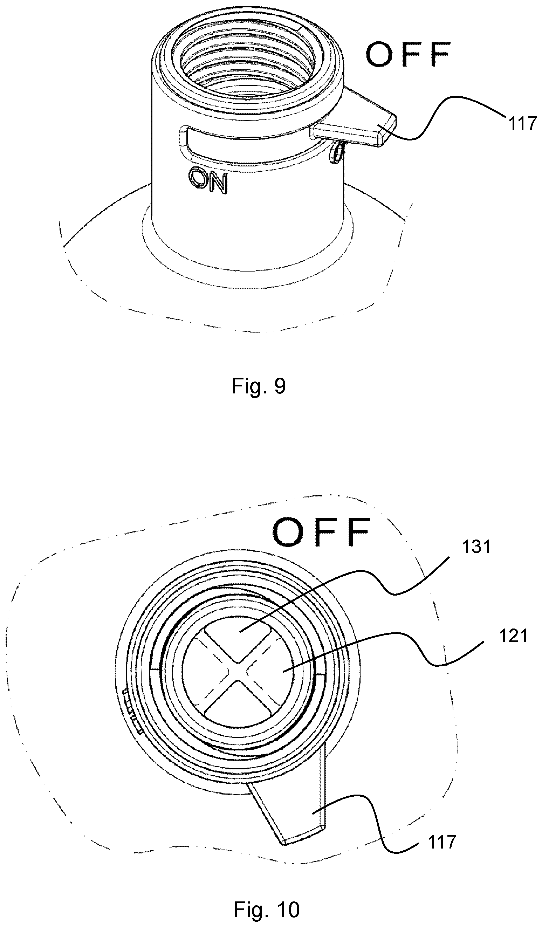

**Figur 9** zeigt einen Ausschnitt einer Gießform, die wie in Bezug auf Figur 4 erläutert einen Einfüllstutzen **120** mit einem Bedienelement **117** aufweist. Das Bedienelement **117** befindet sich hier in einer zweiten Position, welche den Durchfluss von Knochenzement durch den Einfüllstutzen **120** verhindert. Diese zweite Position des Bedienelements **107** ist mit einer Beschriftung "OFF" gekennzeichnet, um einem Benutzer der Gießform zu erkennen zu geben, dass in dieser Konfiguration die Gießform vollständig verschlossen ist, sodass kein Knochenzement durch den Einfüllstutzen **120** fließen kann.

**Figur 10** zeigt einen Ausschnitt einer Gießform gemäß Figur 9, wobei sich das Bedienelement **117** in einer zweiten Position befindet. Hierbei weist die Gießform eine erste Durchführung **131** und eine zweite Durchführung **121,** welche in der zweiten Position des Bedienelements **117** versetzt zueinander, d. h. nicht entlang einer gemeinsamen Achse angeordnet sind. Die erste Durchführung **131** ist dadurch von der zweiten Durchführung **121** isoliert. Somit kann kein Knochenzement durch den Einfüllstutzen **120** gelangen. Weiterhin weist die Gießform einen ersten Vorsprung **132** und einen zweiten Vorsprung **122** auf, die ebenfalls in der zweiten Position des Bedienelements **117** versetzt zueinander angeordnet sind. Durch die Bewegung des ersten Vorsprung **132** im Verhältnis zu dem zweiten Vorsprung **122** wird erstens der Einfüllstutzen **120** für den Durchfluss von Knochenzement gesperrt, und zweitens der noch verformbarer Anguss des Knochenzements abgetrennt. Somit kann die Bildung eines Angusses vermieden oder zumindest deutlich verringert werden.

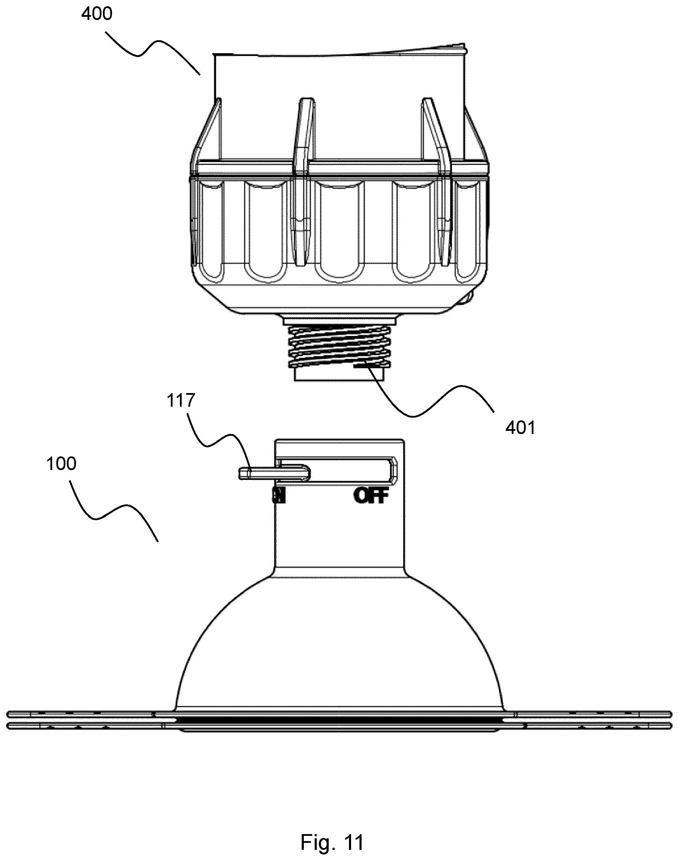

**Figur 11** zeigt eine erfindungsgemäße Gießform **100** mit Bedienelement **117** und einer Austragsvorrichtung **400** für Knochenzement, welche zur Verbindung mit der Gießform **100** angeordnet ist. Die Austragsvorrichtung **400** weist ein Gewinde **401** auf, welches in ein entsprechendes Gewinde am Einfüllstutzen **120** eingreifen kann. Die hier gezeigte Ausführungsform der Gießform **100** ist ausgebildet und eingerichtet, das Bedienelement **117** in eine erste Position zu bewegen, wenn eine Austragsvorrichtung **400** mit einem Gewinde **401** in den Einfüllstutzen **120** der Gießform **100** eingeschraubt ist. Somit wird die Gießform **100** automatisch in einen Zustand gebracht, der die Aufnahme von Knochenzement aus der Austragsvorrichtung **400** in die Gießform **100** erlaubt, wenn die Austragsvorrichtung **400** in die Gießform **100** eingeschraubt wird.

### LISTE DER BEZUGSZEICHEN

- 100: Gießform
- 110: Grundkörper
- 111: erste Schale
- 112: zweite Schale
- 113: erster Steg
- 114: zweiter Steg
- 115: Hohlraum
- 116: Schnittbereich
- 117: Bedienelement
- 118: Entlüftungsöffnung
- 120: Einfüllstutzen
- 121: zweite Durchführung
- 122: zweiter Vorsprung
- 123: Gewinde
- 124: Hals
- 130: Stopfen
- 131: erste Durchführung
- 132: erster Vorsprung
- 133: Schneidkante
- 134: Gelenk
- 200: Knochenzement
- 201: Anguss
- 202: Spacer
- 300: Skalpell
- 400: Austragsvorrichtung
- 401: Gewinde

## Patentansprüche

1. Gießform (100) zur Herstellung eines Acetabulum-Spacers, aufweisend einen Grundkörper (110) mit einer gewölbten ersten Schale (111) und einer gewölbten zweiten Schale (112), einen ersten Steg (113) und einen zweiten Steg (114), einen Einfüllstutzen (120) und eine erste Durchführung (131), wobei die erste Schale (111) gemeinsam mit der zweiten Schale (112) einen Hohlraum (115) definiert, wobei der erste Steg (113) und der zweite Steg (114) benachbart zueinander an dem Grundkörper (110) angeordnet sind und gemeinsam einen Schnittbereich (116) des Grundkörpers (110) definieren, wobei der Schnittbereich (116) zwischen dem ersten Steg (113) und dem zweiten Steg (114) angeordnet ist, und wobei die erste Durchführung (131) ausgebildet ist, den Austritt von überschüssigem Knochenzement (200) aus dem Hohlraum (115) der Gießform (100) zu ermöglichen.

2. Gießform nach Anspruch 1, wobei der Grundkörper (110) eine erste Wandstärke aufweist, welche dicker als eine zweite Wandstärke des Schnittbereichs (116) ist.

3. Gießform nach einem der vorangehenden Ansprüche, wobei der erste Steg (113) und der zweite Steg (114) ausgebildet sind, ein Skalpell zum Durchtrennen der Gießform (100) entlang des Schnittbereichs (116) zu führen.

4. Gießform nach einem der vorangehenden Ansprüche, wobei der erste Steg (113) und/oder der zweite Steg (114) eine Lasche aufweist, um ein manuelles Auseinanderziehen der ersten Schale (111) und der zweiten Schale (112) zu erleichtern.

5. Gießform nach einem der vorangehenden Ansprüche, wobei der erste Steg (113) und/oder der zweite Steg (114) sich um mindestens 2 mm, weiter bevorzugt mindestens 3, 4, 5 oder 6 mm, von dem Grundkörper (110) nach außen erstreckt.

6. Gießform nach einem der vorangehenden Ansprüche, wobei sich der erste Steg (113) und/oder der zweite Steg (114) sich umlaufend um mindestens die Hälfte eines Umfangs des Grundkörpers (110) erstreckt.

7. Gießform nach einem der vorangehenden Ansprüche, weiterhin aufweisend ein Mittel zum Abtrennen eines Angusses, wobei das Mittel bevorzugt ein bewegliches Element aufweist, wobei das bewegliche Element weiter bevorzugt ausgewählt ist aus einer Durchführung (121, 131) und einem Vorsprung (122, 132).

8. Gießform nach einem der vorangehenden Ansprüche, weiterhin aufweisend einen Stopfen (130) zum Verschließen des Einfüllstutzens (120), wobei die erste Durchführung (131) bevorzugt in dem Stopfen (130) angeordnet ist.

9. Gießform nach Anspruch 8, wobei die Gießform (130) ausgebildet und eingerichtet ist, durch eine Drehbewegung des Stopfens (130) relativ zum Einfüllstutzen (120) einen Anguss (201) abzutrennen.

10. Gießform nach einem der Ansprüche 8 oder 9, wobei der Stopfen (130) einen ersten Vorsprung (132) zur Abtrennung eines Angusses (201) aufweist, wobei der erste Vorsprung (132) bevorzugt an der ersten Durchführung (131) angeordnet ist.

11. Gießform nach Anspruch 10, wobei der erste Vorsprung (132) eine angeschrägte Schneidkante (133) aufweist.

12. Gießform nach einem der vorangehenden Ansprüche 8 bis 11, wobei der Einfüllstutzen (120) einen zweiten Vorsprung (122) zur Abtrennung eines Angusses (201) aufweist, wobei die Gießform bevorzugt weiterhin einen ersten Vorsprung (132) gemäß Anspruch 10 aufweist, und wobei die Gießform bevorzugt ausgebildet und eingerichtet ist, durch eine Drehbewegung des Stopfens (130) relativ zum Einfüllstutzen (120) einen Anguss (201) abzutrennen, wenn der erste Vorsprung (132) relativ zu dem zweiten Vorsprung (122) gedreht wird.

13. Gießform nach einem der Ansprüche 8 bis 12, wobei die erste Durchführung (131) exzentrisch in dem Stopfen (130) angeordnet ist.

14. Gießform nach einem der vorangehenden Ansprüche 8 bis 13, wobei der Einfüllstutzen (120) weiterhin eine zweite Durchführung (121) aufweist, wobei die Gießform ausgebildet und eingerichtet ist, in einer ersten Drehposition des Stopfens die erste Durchführung (131) entlang einer gemeinsamen Achse mit der zweiten Durchführung (121) anzuordnen, und in einer zweiten Drehposition des Stopfens die erste Durchführung (131) außerhalb einer gemeinsamen Achse mit der zweiten Durchführung (121) anzuordnen.

15. Gießform nach einem der vorangehenden Ansprüche, weiterhin aufweisend ein von außerhalb der Gießform zugängliches Bedienelement (117), welches ausgebildet und eingerichtet ist, einen Anguss (201) abzutrennen.

16. Gießform nach einem der vorangehenden Ansprüche, weiterhin aufweisend eine Entlüftungsöffnung (118), wobei die Entlüftungsöffnung (118) bevorzugt zwischen der ersten Schale (111) und der zweiten Schale (112) angeordnet ist.

17. Verwendung einer Gießform nach einem der vorangehenden Ansprüche zur Herstellung eines Acetabulum-Spacers.

18. Verfahren zur Herstellung eines Acetabulum-Spacers, aufweisend die nachfolgenden Schritte,
Bereitstellen einer Gießform nach einem der Ansprüche 1 bis 16;
Befüllen der Gießform mit Knochenzement;
Härten des Knochenzements in der Gießform;
gegebenenfalls Entfernen eines aus dem Knochenzement gebildeten Angusses;
Auftrennen des Grundkörpers (110) der Gießform im Schnittbereich (116);
Trennen der ersten Schale (111) von der zweiten Schale (112); und
Entnahme des hergestellten Acetabulum-Spacers aus der aufgetrennten Gießform.
